(19) Europäisches Patentamt
European Patent Office
Office européen des brevets

(11) Veröffentlichungsnummer: **0 080 674**
**B1**

(12)
# EUROPÄISCHE PATENTSCHRIFT

(45) Veröffentlichungstag der Patentschrift: **20.03.85**

(51) Int. Cl.⁴: **C 07 J 41/00,** A 61 K 31/705 // C07J19/00

(21) Anmeldenummer: **82110728.1**

(22) Anmeldetag: **20.11.82**

(54) **Neue Oxime von 3‴-Dehydro-cardenolid-tridigitoxosiden, Verfahren zu ihrer Herstellung und ihre Verwendung als Arzneimittel.**

(30) Priorität: **26.11.81 DE 3146898**

(43) Veröffentlichungstag der Anmeldung: **08.06.83 Patentblatt 83/23**

(45) Bekanntmachung des Hinweises auf die Patenterteilung: **20.03.85 Patentblatt 85/12**

(84) Benannte Vertragsstaaten: **AT BE CH DE FR GB IT LI LU NL SE**

(56) Entgegenhaltungen: **EP - A - 0 021 244**

(73) Patentinhaber: **Boehringer Mannheim GmbH, *Sandhoferstrasse 116, D-6800 Mannheim 31 (DE)***

(72) Erfinder: **Schaumann, Wolfgang, Prof. Dr. med., Mönchhofstrasse 58, D-6900 Heidelberg (DE)**
Erfinder: **Kaiser, Fritz, Dr. rer. nat., Hans-Holbein-Strasse 20, D-6840 Lampertheim (DE)**
Erfinder: **Voigtländer, Wolfgang, Dr. rer. nat., Haselnussweg 30, D-6940 Weinheim (DE)**
Erfinder: **Hoyer, Edgar, In den alten Wiesen 55, D-6800 Mannheim (DE)**
Erfinder: **Neubert, Peter, Dr. rer. nat., Telemannstrasse 5, d-6940 Weinheim (DE)**

**EP 0 080 674 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue Verbindungen der Formel I

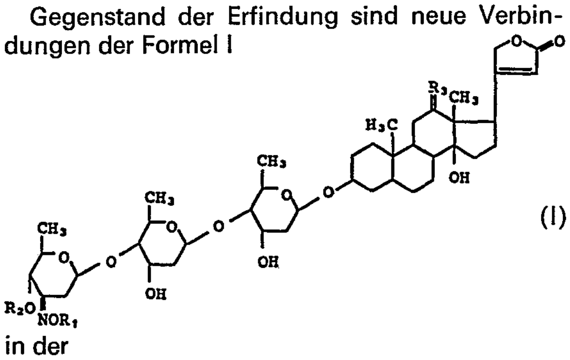

(I)

in der

$R_1$ Wasserstoff oder eine Alkylgruppe mit 1 bis 3 Kohlenstoffatomen,

$R_2$ Wasserstoff, einen Alkanoylrest mit 1 bis 3 Kohlenstoffatomen oder eine Alkylgruppe mit 1 bis 3 Kohlenstoffatomen,

$R_3$ 2 Wasserstoffatome, die Gruppe 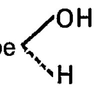 oder die Oximinogruppe $=NOR_1$, worin $R_1$ die angegebene Bedeutung besitzt,

darstellen

und Verfahren zu ihrer Herstellung sowie ihre Verwendung zur Herstellung von Arzneimitteln gegen Herzinsuffizienz.

Unter einem Alkanoylrest mit 1 bis 3 Kohlenstoffatomen ist vorzugsweise der Acetylrest zu verstehen. Ein Alkylrest mit 1 bis 3 Kohlenstoffatomen ist vorzugsweise die Methylgruppe.

Die in der Therapie der Herzinsuffizienz hauptsächlich verwendeten Digitalisglykoside Digitoxin und Digoxin mit ihren Derivaten, z.B. Acetyldigoxin, Methyldigoxin, lassen für die Sicherheit ihrer Anwendung noch Wünsche offen: Digoxin und Derivate werden überwiegend durch die Nieren ausgeschieden und können deshalb bei Patienten mit verringerter Nierenfunktion zu Intoxikationen führen.

Digitoxin ist das Glykosid mit der längsten Verweildauer im Organismus, weshalb etwa auftretende Intoxikationen (z.B. bei Überdosierung) nur extrem langsam wieder abklingen können.

Es wurde nun gefunden, dass die erfindungsgemässen Oximinoderivate von 3'''-Dehydrocardenolidtridigitoxosiden eine ideale Mittelstellung einnehmen, indem sie überwiegend extrarenal ausgeschieden werden und somit bei verminderter Nierenfunktion weniger gefährlich sind und zudem erheblich schneller eliminiert werden als Digitoxin, und zwar mit Eliminationszeiten ähnlich der des Digoxins.

Die neuen Verbindungen der Formel I können wie folgt hergestellt werden.

Verbindungen der Formel II

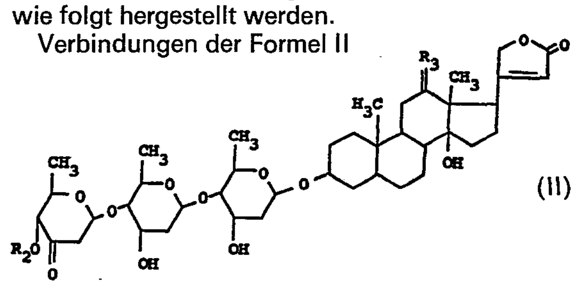

(II)

in der

$R_2$ Wasserstoff, einen Alkanoylrest mit 1 bis 3 Kohlenstoffatomen oder eine Alkylgruppe mit 1 bis 3 Kohlenstoffatomen,

$R_3$ 2 Wasserstoffatome, die Gruppe 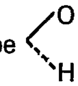 oder ein Sauerstoffatom bedeutet,

werden in an sich bekannter Weise mit Hydroxylaminen der Formel III

$$R_1-O-NH_2 \qquad (III)$$

in der $R_1$ Wasserstoff oder eine Alkylgruppe mit 1 bis 3 Kohlenstoffatomen darstellt oder ihren Salzen mit geeigneten Säuren, in polaren Lösungsmitteln, wie Wasser, Alkoholen, wie z.B. Methanol, Ethanol oder i-Propanol, tert.-Aminen, wie Pyridin oder Triethylamin, oder Dimethylformamid bei Temperaturen von 20°C bis zum Siedepunkt des Lösungsmittels gegebenenfalls unter Zusatz von Basen, wie tert.-Aminen, Alkalihydroxyden oder -carbonaten, umgesetzt.

Die Aufarbeitung und Reinigung der Endprodukte erfolgt nach üblichen Methoden u.a. durch chromatographische Verfahren oder durch multiplikative Verteilung und Kristallisation.

Identität und Reinheit der erhaltenen Verbindungen wurden durch Dünnschichtchromatogramm überprüft. Dabei wurden DC-Fertigplatten (Merck Kieselgel 60/F 254, Imprägnierung 20% Formamid in Aceton) eingesetzt und mit Fliessmittel Xylol/Methylethylketon 2:3 + 5% Formamid entwickelt. Die fertigen Chromatogramme wurden mit Trichloressigsäure/Chloramin-Reagens besprüht und die Substanzen durch ihre Fluoreszenzen im langwelligen UV ($\lambda$ = 360 nm) ermittelt. Die Laufstrecken (R) im Chromatogramm wurden jeweils auf einen mitgeführten Standard bezogen. Dabei bedeutet $R_{Dt}$ den auf die Laufstrecke von 3'''-Dehydrodigitoxin bezogenen R-Wert, $R_D$ den auf die Laufstrecke von 3''',12-Didehydrodigoxin bezogenen R-Wert und $R_{Dg}$ den auf die Laufstrekke von 3'''-Dehydrodigoxin bezogenen R-Wert.

Die erfindungsgemässen Cardenolidglykoside können in Einzeldosen von 0,05 bis 1,0 mg 1- bis 4mal täglich appliziert werden. Die Applikation erfolgt vorzugsweise oral, jedoch ist auch eine parenterale Applikation ohne weiteres möglich.

Als orale Darreichungsform werden bevorzugt Tabletten, aber auch Steckkapseln und Weichgelatinekapseln verwendet. Zur individuellen Dosierungseinnahme z.B. für Kinder ist die Zubereitung als Liquidum geeignet. Für die Notfall- und Stationärbehandlung erfolgt die Anwendung durch Injektion entsprechender Lösungen.

Zur Herstellung von Tabletten oder Steckkapseln zur oralen Darreichung wird der Wirkstoff mit üblichen Hilfsstoffen, wie Lactose und Stärke homogen gemischt, wobei wegen der geringen Einzeldosierung die Herstellung einer Vormischung bevorzugt wird. Die Wirkstoff/Hilfsstoff-Mischung kann durch Auswahl geeigneter Hilfsstoffe als trockene Pulvermasse oder durch Granulation mit Bindemitteln, wie Stärkekleister oder Polyvinylpyrrolidon als Granulat in Steckkapseln abgefüllt oder nach weiterer Zumischung üblicher

Sprengmittel und Gleitmittel zu Tabletten verpresst werden.

Trägerstoffe für Weichgelatinekapseln können übliche Glycerinfettsäureester sein, aber auch Polyethylenglykole als Lösungsmittel für den Wirkstoff. Für eine Liquidum- oder Ampullenform können als Lösungsmittel Ethanol oder mehrwertige Alkohole gegebenenfalls unter Zusatz von Wasser und anderen üblichen Hilfsstoffen verwendet werden.

Die Vorteile der erfindungsgemässen Verbindungen gegenüber Digoxin und Digitoxin, das heisst die Verbindung von rascher Ausscheidung mit hoher Ausscheidungsrate über Galle/Faeces ist in dem folgenden Versuchsprotokoll dargestellt.

*Versuchsprotokoll*

Je 4 Katzen erhielten je eine intravenöse Dosis von 20 µg/kg eines der in der folgenden Tabelle genannten Glykoside. Die Glykoside waren nach der Methode Haberland und Maerten* – Digoxin nach der Methode von Wartburg** – mit Tritium markiert.

In den nach 2 und 7 d getrennt gesammelten Urin- und Faeces-Portionen wurde die Radioaktivität bestimmt.

Die in der Tabelle zusammengestellten Werte geben die Ausscheidungsgeschwindigkeit (Spalte I) und den Anteil der Ausscheidung im Urin wieder (Spalte II).

Es bedeuten:

Spalte I: ausgeschiedene Menge in Harn + Faeces nach 2 d in Prozent der Gesamtausscheidung nach 7 d.

Spalte II: prozentualer Anteil der Ausscheidung im Harn nach 7 d bezogen auf die Gesamtausscheidung in Harn + Faeces nach 7 d.

| Glykosid | I | II |
|---|---|---|
| Digoxin | 53 | 44 |
| Digitoxin | 20 | 16 |
| 3'''-Dehydrodigitoxinoxim | 53 | 14 |
| 3'''-Dehydrodigitoxinmethyloxim | 54 | 21 |
| 3'''-Dehydrodigitoxinethyloxim | 52 | 20 |
| 3''',12-Didehydrodigoxin-dimethyloxim | 45 | 21 |

* DE-A Nr. 1959064

** „Biochem. Pharm.", *14*, 1883 (1965)

*Beispiel 1:*

*3'''-Dehydrodigitoxinoxim*

2 g 3'''-Dehydrodigitoxin in 20 ml Pyridin und 40 ml Ethanol gelöst, werden nach Zugabe von 1 g Hydroxylaminhydrochlorid 1 h unter Rückfluss zum Sieden erhitzt, mit Wasser verdünnt, mit Chloroform ausgeschüttelt, die Chloroformphasen mit 2N-Schwefelsäure, Sodalösung und Wasser gewaschen, über Natriumsulfat getrocknet und im Vakuum eingeengt. Das Rohprodukt wird zweimal aus Chloroform/Methanol/Ether kristallisiert und liefert 1,2 g 3'''-Dehydrodigitoxinoxim.

Smp.: 196-199°C

$R_{Dt}$: 0,90

*Beispiel 2:*

*3'''-Dehydrodigitoxinmethyloxim*

1 g 3'''-Dehydrodigitoxin in 20 ml Pyridin gelöst, wird nach Zugabe von 500 mg O-Methylhydroxylaminhydrochlorid 1 d bei Raumtemperatur stehen gelassen und wie unter Beispiel 1 beschrieben aufgearbeitet. Das Rohprodukt wird aus Aceton kristallisiert und liefert 870 mg 3'''-Dehydrodigitoxinmethyloxim.

Smp.: 246-249°C

$R_{Dt}$: 1,18

*Beispiel 3:*

*3'''-Dehydrodigitoxinethyloxim*

1 g 3'''-Dehydrodigitoxin in 10 ml Pyridin gelöst, wird nach Zugabe von 500 mg O-Ethylhydroxylaminhydrochlorid 3 d bei Raumtemperatur stehen gelassen, mit 100 ml Wasser verdünnt, mit Chloroform ausgeschüttelt und die Chloroformphasen, nach Waschen mit 2N-Schwefelsäure, Sodalösung und Wasser, über Natriumsulfat getrocknet und im Vakuum eingeengt. Das Rohprodukt wird mit Cyclohexan/Essigester 3:1 über eine Cellulosesäule (mit Formamid imprägniert) getrennt. Die chromatographisch einheitlichen Fraktionen liefern nach Kristallisation aus Aceton 450 mg 3'''-Dehydrodigitoxinethyloxim.

Smp.: 261-265°C

$R_{Dt}$: 1,28

*Beispiel 4:*

*3''',12-Didehydrodigoxindioxim*

1 g 3''',12-Didehydrodigoxin in 20 ml Pyridin und 20 ml Ethanol gelöst, wird nach Zugabe von 300 mg Hydroxylaminhydrochlorid 3 h unter Rückfluss zum Sieden erhitzt, wie unter Beispiel 1 beschrieben aufgearbeitet und das Rohprodukt in Chloroform/Methanol 1:1 gelöst, mit Tierkohle versetzt, über Kieselgel filtriert, eingeengt und aus Aceton/Ether kristallisiert. Ausbeute: 620 mg 3''',12-Didehydrodigoxindioxim.

Smp.: 168-172°C

$R_D$: 0,69

Das als Ausgangsprodukt verwendete 3''',12-Didehydrodigoxin ist neu und wird wie folgt hergestellt.

10 g Digoxin in 400 ml Eisessig gelöst werden bei Raumtemperatur innerhalb 4 h portionsweise mit 160 ml 2% Chromtrioxyd/Eisessig-Lösung versetzt. Anschliessend wird mit 1,5 l Chloroform verdünnt, mit 2N-Schwefelsäure (zweimal 250 ml) und Wasser (zweimal 250 ml) gewaschen, über Natriumsulfat getrocknet und im Vakuum eingeengt. Das Rohprodukt wird mit Chloroform + 2% Methanol über Kieselgel fraktioniert. Die chromatographisch einheitlichen Fraktionen liefern nach Kristallisation aus Chloroform/Ether 6,5 g 3''',12-Didehydrodigoxin.

Smp.: 206-210°C

*Beispiel 5:*

*3''',12-Didehydrodigoxindimethyloxim*

1 g 3''',12-Didehydrodigoxin in 20 ml Pyridin und 20 ml Ethanol gelöst, wird nach Zugabe von 500 mg O-Methylhydroxylaminhydrochlorid, wie unter Beispiel 4 beschrieben, umgesetzt und aufgearbeitet. Das Rohprodukt wird mit Cyclohexan/Essigester 2:1 über eine Cellulosesäule (mit Formamid imprägniert) getrennt. Die chromatographisch einheitlichen Fraktionen liefern nach Kristallisation aus Aceton/Ether 450 mg 3''',12-Didehydrodigoxindimethyloxim.

Smp.: 239-243°C

$R_D$: 1,35

*Beispiel 6:*

*3''',12-Didehydrodigoxindiethyloxim*

1 g 3''',12-Didehydrodigoxin in 10 ml Pyridin gelöst, wird nach Zugabe von 500 mg O-Ethylhydroxylaminhydrochlorid 3 d bei Raumtemperatur stehen gelassen, wie unter Beispiel 3 beschrieben aufgearbeitet und das Rohprodukt mit Heptan/Methylethylketon 3:1 über eine Cellulosesäule (mit Formamid imprägniert) getrennt. Die chromatographisch einheitlichen Fraktionen liefern nach Kristallisation aus Aceton 430 mg 3''',12-Didehydrodigoxindiethyloxim.

Smp.: 241-245°C

$R_D$: 1,43

*Beispiel 7:*

*3'''-Dehydrodigoxinmethyloxim*

1 g 3'''-Dehydrodigoxin in 20 ml Pyridin gelöst, wird nach Zugabe von 120 mg O-Methylhydroxylaminhydrochlorid 30 min bei Raumtemperatur stehen gelassen und wie unter Beispiel 1 beschrieben aufgearbeitet. Das Rohprodukt wird aus Aceton kristallisiert und liefert 820 mg 3'''-Dehydrodigoxinmethyloxim.

Smp.: 251-254°C

$R_{Dg}$: 1,43

*Beispiel 8:*

*4'''-Acetyl-3'''-dehydrodigitoxinoxim*

2 g 3'''-Dehydrodigitoxinoxim in 20 ml Dimethylformamid gelöst, werden nach Zugabe von 400 mg Triethylendiamin und 0,26 ml Essigsäureanhydrid 20 h bei Raumtemperatur stehen gelassen, mit 150 ml Wasser verdünnt, mit Chloroform ausgeschüttelt und die Chloroformphasen im Vakuum eingeengt. Das Rohprodukt wird mit Cyclohexan/Essigester 3:1 über eine Cellulosesäule (mit Formamid imprägniert) getrennt. Die chromatographisch einheitlichen Fraktionen liefern nach Kristallisation aus Essigester 620 mg 4'''-Acetyl-3'''-dehydrodigitoxinoxim.

Smp.: 202-205°C

$R_{Dt}$: 1,09

*Beispiel 9:*

*4'''-Acetyl-3'''-dehydrodigitoxinmethyloxim*

2 g 3'''-Dehydrodigitoxinmethyloxim in 20 ml Dimethylformamid gelöst, werden nach Zugabe von 400 mg Triethylendiamin und 0,26 ml Essigsäureanhydrid, wie unter Beispiel 8 beschrieben umgesetzt und aufgearbeitet. Das Rohprodukt wird mit Heptan/Methylethylketon 2:1 über eine Cellulosesäule (mit Formamid imprägniert) getrennt. Die chromatographisch einheitlichen Fraktionen liefern nach Kristallisation aus Aceton 1,2 g 4'''-Acetyl-3'''-dehydrodigitoxinmethyloxim.

Smp.: 233-236°C

$R_{Dt}$: 1,32

*Beispiel 10:*

*4'''-Methyl-3'''-dehydrodigitoxinoxim*

1,8 g 4'''-Methyl-3'''-dehydrodigitoxin in 72 ml Pyridin gelöst, werden nach Zugabe von 720 mg Hydroxylaminhydrochlorid 2 h auf 100°C erwärmt, mit 900 ml Wasser verdünnt, mit Chloroform ausgeschüttelt, die Chloroformphasen mit Wasser gewaschen, über Natriumsulfat getrocknet und im Vakuum eingeengt. Das Rohprodukt wird mit Cyclohexan/Essigester 2:1 über eine Cellulosesäule (mit Formamid imprägniert) getrennt. Die chromatographisch einheitlichen Fraktionen liefern nach Kristallisation aus Aceton/Ether 1,05 g 4'''-Methyl-3'''-dehydrodigitoxinoxim.

Smp.: 193-195°C

$R_{Dt}$: 1,11

Das als Ausgangsprodukt verwendete 4'''-Methyl-3'''-dehydrodigitoxin ist neu und wird wie folgt hergestellt.

In ein Gemisch von 8 ml Pyridin und 150 ml Methylenchlorid werden unter Rühren bei Raumtemperatur 6 g Chromtrioxyd gegeben und 15 min bei Raumtemperatur gerührt. Eine Lösung von 8 g 4'''-Methyldigitoxin in 10 ml Pyridin und 100 ml Methylenchlorid wird langsam zugegeben, 15 min bei Raumtemperatur gerührt, 1 h unter Rückfluss zum Sieden erhitzt, mit 500 ml Wasser verdünnt, mit Chloroform ausgeschüttelt, die Chloroformphasen mit Natriumbicarbonatlösung (5%) und Wasser gewaschen, über Natriumsulfat getrocknet und im Vakuum eingeengt. Das Rohprodukt wird mit Heptan/Methylethylketon 2:1 über eine Cellulosesäule (mit Formamid imprägniert) getrennt. Die chromatographisch einheitlichen Fraktionen liefern nach Kristallisation aus Chloroform/Ether 4,2 g 4'''-Methyl-3'''-dehydrodigitoxin.

Smp.: 213-217°C

*Beispiel 11:*

*4'''-Methyl-3'''-dehydrodigitoxinmethyloxim*

1 g 4'''-Methyl-3'''-dehydrodigitoxin in 5 ml Pyridin gelöst, wird nach Zugabe von 250 mg O-Methylhydroxylaminhydrochlorid, wie unter Beispiel 2 beschrieben, umgesetzt und aufgearbeitet. Das Rohprodukt wird aus Methanol/Wasser 5:2 kristallisiert und liefert 720 mg 4'''-Methyl-3'''-dehydrodigitoxinmethyloxim.

Smp.: 171-175°C

$R_{Dt}$: 1,36

*Beispiel 12:*

*4'''-Methyl-3'''-dehydrodigoxinoxim*

1 g 4'''-Methyl-3'''-dehydrodigoxin in 10 ml Pyridin und 20 ml Ethanol gelöst, wird nach Zugabe von 500 mg Hydroxylaminhydrochlorid, wie unter Beispiel 1 beschrieben, umgesetzt und aufgearbeitet. Das Rohprodukt liefert nach Kristallisation aus Essigester/Ether 680 mg 4'''-Methyl-3'''-dehydrodigoxinoxim.

Smp.: 159-163°C

$R_{Dg}$: 1,74

*Beispiel 13:*

*4'''-Acetyl-3''',12-didehydrodigoxindimethyloxim*

1 g 3''',12-Didehydrodigoxindimethyloxim in 10 ml Dimethylformamid gelöst, wird nach Zugabe von 200 mg Triethylendiamin und 0,20 ml Essigsäureanhydrid wie unter Beispiel 8 beschrieben, umgesetzt und aufgearbeitet. Das Rohprodukt wird mit Cyclohexan/Essigester 4:1 über eine Cellulosesäule (mit Formamid imprägniert) getrennt. Die chromatographisch einheitlichen Fraktionen liefern nach Kristallisation aus Ether/Petrolether 490 mg 4'''-Acetyl-3''',12-didehydrodigoxindimethyloxim.

Smp.: 126-130°C

$R_D$: 1,86

*Beispiel 14:*

*4'''-Methyl-3''',12-didehydrodigoxindioxim*

1 g 4'''-Methyl-3''',12-didehydrodigoxin in 20 ml Pyridin und 20 ml Ethanol gelöst, wird nach Zugabe von 300 mg Hydroxylaminhydrochlorid 3 h unter Rückfluss zum Sieden erhitzt und wie unter Beispiel 1 beschrieben aufgearbeitet. Das Rohprodukt liefert nach Kristallisation aus Methanol/Wasser 630 mg 4'''-Methyl-3''',12-didehydrodigoxindioxim.

Smp.: 154-158°C

$R_D$: 1,13

Das als Ausgangsprodukt verwendete 4'''-Methyl-3''',12-didehydrodigoxin ist neu und wird wie folgt hergestellt.

10 g 4'''-Methyldigoxin in 400 ml Eisessig gelöst, werden bei Raumtemperatur innerhalb 4 h portionsweise mit 160 ml 2% Chromtrioxyd/Eisessig-Lösung versetzt. Anschliessend wird mit 1,5 l Chloroform verdünnt, mit 2N-Schwefelsäure (zweimal 250 ml) und Wasser (zweimal 250 ml) gewaschen, über Natriumsulfat getrocknet und im Vakuum eingeengt. Das Rohprodukt wird mit Chloroform + 1% Methanol über Kieselgel fraktioniert. Die chromatographisch einheitlichen Fraktionen liefern nach Kristallisation aus Chloroform/Methanol/Ether 5,9 g 4'''-Methyl-3''',12-didehydrodigoxin.

Smp.: 209-212°C

*Beispiel 15:*

*4'''-Methyl-3''',12-didehydrodigoxindimethyloxim*

1 g 4'''-Methyl-3''',12-didehydrodigoxin in 20 ml Pyridin und 20 ml Ethanol gelöst, wird nach Zugabe von 500 mg O-Methylhydroxylaminhydrochlorid wie unter Beispiel 14 beschrieben, umgesetzt und aufgearbeitet. Das Rohprodukt liefert nach Kristallisation aus Aceton/Ether/Petrolether 520 mg 4'''-Methyl-3''',12-didehydrodigoxindimethyloxim.

Smp.: 89-93°C

$R_D$: 1,87

**Patentansprüche**

1. 3'''-Dehydrocardenolidtridigitoxosidoxime der Formel (I)

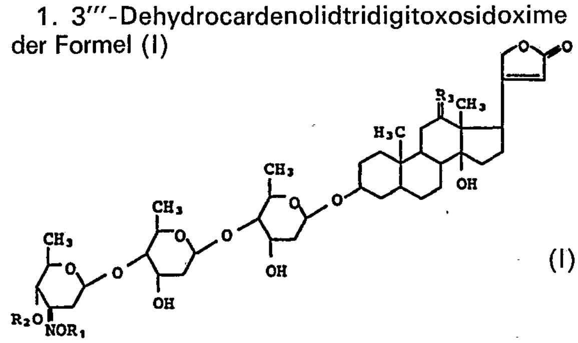

(I)

in der

$R_1$ Wasserstoff oder eine Alkylgruppe mit 1 bis 3 Kohlenstoffatomen,

$R_2$ Wasserstoff, einen Alkanoylrest mit 1 bis 3 Kohlenstoffatomen oder eine Alkylgruppe mit 1 bis 3 Kohlenstoffatomen,

$R_3$ 2 Wasserstoffatome, die Gruppe 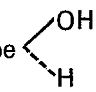 oder die Oximinogruppe $=NOR_1$, worin $R_1$ die angegebene Bedeutung besitzt,

darstellen.

2. Verfahren zur Herstellung von 3'''-Dehydrocardenolidtridigitoxosidoxime der Formel (I)

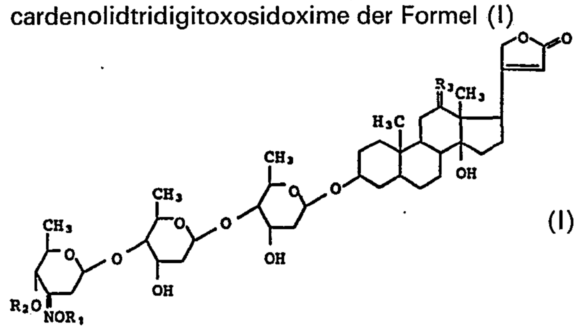

(I)

in der

$R_1$ Wasserstoff oder eine Alkylgruppe mit 1 bis 3 Kohlenstoffatomen,

$R_2$ Wasserstoff, einen Alkanoylrest mit 1 bis 3 Kohlenstoffatomen oder eine Alkylgruppe mit 1 bis 3 Kohlenstoffatomen,

$R_3$ 2 Wasserstoffatome, die Gruppe 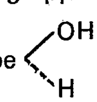 oder die Oximinogruppe $=NOR_1$, worin $R_1$ die angegebene Bedeutung besitzt,

darstellen, dadurch gekennzeichnet, dass man 3'''-Dehydrocardenolidtridigitoxoside der Formel (II)

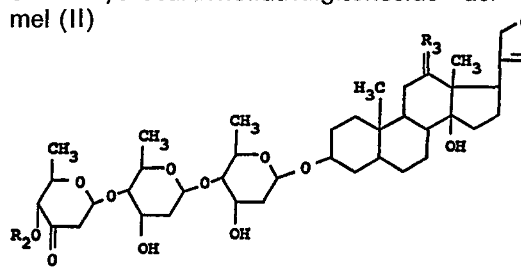

(II)

in der

$R_2$ Wasserstoff, einen Alkanoylrest mit 1 bis 3 Kohlenstoffatomen oder eine Alkylgruppe mit 1 bis 3 Kohlenstoffatomen,

$R_3$ 2 Wasserstoffatome, die Gruppe 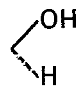 oder ein Sauerstoffatom bedeutet,

werden in an sich bekannter Weise mit Hydroxylaminen der Formel (III)

$$R_1-O-NH_2 \qquad (III)$$

in der $R_1$ Wasserstoff oder eine Alkylgruppe mit 1 bis 3 Kohlenstoffatomen darstellt oder ihren Salzen mit geeigneten Säuren

in polaren Lösungsmitteln, bei Temperaturen von 20°C bis zum Siedepunkt des Lösungsmittels gegebenenfalls unter Zusatz von Basen, umsetzt.

3. Arzneimittel gekennzeichnet durch einen Gehalt an 3'''-Dehydrocardenolidtridigitoxosidoximen der Formel (I).

4. Verbindungen gemäss Anspruch 1 zur Verwendung bei der Bekämpfung der Herzinsuffizenz.

## Claims

1. 3'''-Dehydrocardenolide tridigitoxoside oximes of the Formula (I)

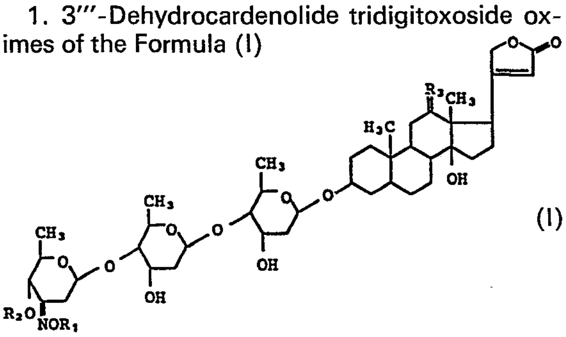

(I)

in which

$R_1$ represents hydrogen or an alkyl group with 1-3 carbon atoms,

$R_2$ hydrogen, an alkanoyl radical with 1-3 carbon atoms or an alkyl group with 1-3 carbon atoms,

$R_3$ 2 hydrogen atoms, the group 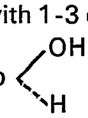 or the oximino group $=NOR_1$, wherein $R_1$ possesses the given meaning.

2. Process for the preparation of 3'''-dehydrocardenolide tridigitoxoside oximes of the Formula (I)

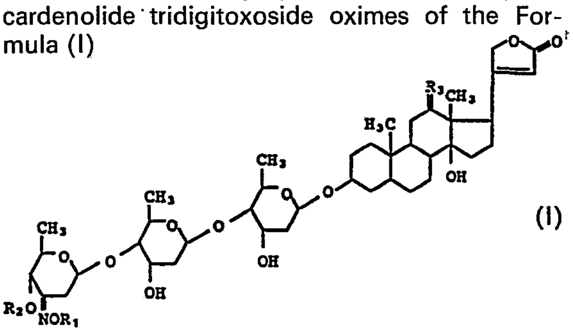

(I)

in which

$R_1$ represents hydrogen or an alkyl group with 1-3 carbon atoms,

$R_2$ hydrogen, an alkanoyl radical with 1-3 carbon atoms or an alkyl group with 1-3 carbon atoms,

$R_3$ 2 hydrogen atoms, the group 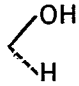 or the oximino group $=NOR_1$, wherein $R_1$ possesses the given meaning,

characterised in that one reacts 3'''-dehydrocardenolide tridigitoxosides of the Formula (II)

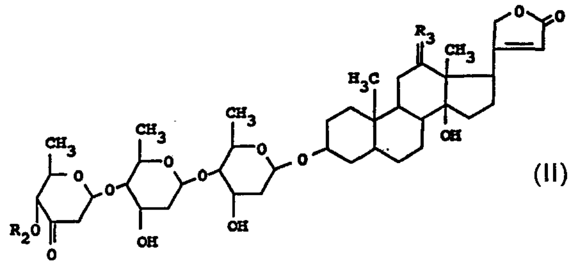

(II)

in which

$R_2$ signifies hydrogen, an alkanoyl radical with 1-3 carbon atoms or an alkyl group with 1-3 carbon atoms,

$R_3$ 2 hydrogen atoms, the group 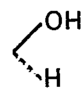 or an oxygen atom,

in *per se* known manner with hydroxylamines of the Formula (III)

$$R_1-O-NH_2 \qquad (III)$$

in which $R_1$ represents hydrogen or an alkyl group with 1-3 carbon atoms or their salts with appropriate acids

in polar solvents at temperatures of 20°C to the boiling point of the solvent, optionally with the addition of bases.

3. Medicaments, characterised by a content of 3'''-dehydrocardenolide tridigitoxoside oximes of the Formula (I).

4. Compounds according to Claim 1 for use in the combating of heart insufficiency.

## Revendications

1. Oximes 3'''-déhydrocardénolidetridigitoxosides de formule (I)

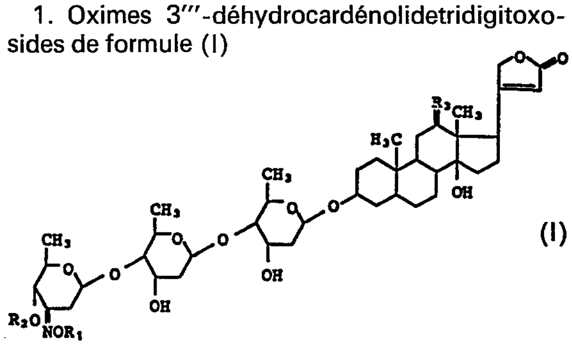

(I)

dans laquelle

$R_1$ est de l'hydrogène ou un groupe alkyle ayant de 1 à 3 atomes de carbone,

$R_2$ est de l'hydrogène, un reste alcanoyle ayant de 1 à 3 atomes de carbone ou un groupe alkyle ayant de 1 à 3 atomes de carbone,

$R_3$ représente 2 atomes d'hydrogène, le groupe

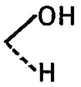 ou le groupe oximino $=NOR_1$, où $R_1$ a la signification donnée ci-dessus.

2. Procédé de préparation d'oximes 3'''-déhydrocardénolidetridigitoxosides de formule (I)

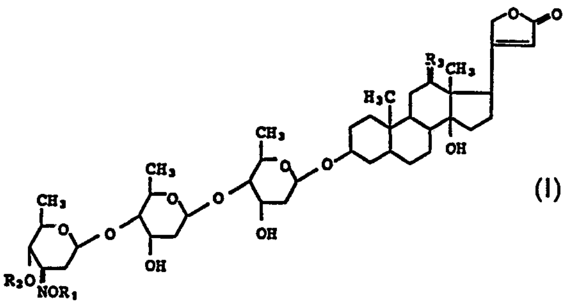

(I)

dans laquelle

$R_1$ est de l'hydrogène ou un groupe alkyle ayant de 1 à 3 atomes de carbone,

$R_2$ est de l'hydrogène, un reste alcanoyle ayant de 1 à 3 atomes de carbone ou un groupe alkyle ayant de 1 à 3 atomes de carbone,

$R_3$ représente 2 atomes de carbone, le groupe

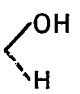 ou le groupe oximino $=NOR_1$, où $R_1$ a la signification indiquée,

caractérisé en ce qu'on fait réagir un 3'''-déhydrocardénolidetridigitoxoside de formule (II)

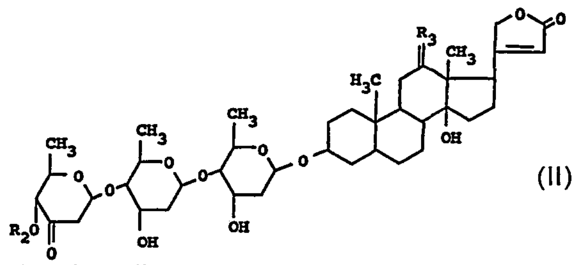

(II)

dans laquelle

$R_2$ est de l'hydrogène, un reste alcanoyle ayant de 1 à 3 atomes de carbone ou un groupe alkyle ayant de 1 à 3 atomes de carbone,

$R_3$ représente 2 atomes de carbone, le groupe

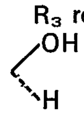 ou un atome d'oxygène,

de façon en soi connue, avec une hydroxylamine de formule (III)

$$R_1-O-NH_2 \qquad (III)$$

dans laquelle $R_1$ est de l'hydrogène ou un groupe alkyle ayant de 1 à 3 atomes de carbone ou leurs sels avec des acides appropriés,

dans un solvant polaire à une température comprise entre 20° C et le point d'ébullition du solvant, éventuellement en ajoutant une base.

3. Médicament, caractérisé en ce qu'il a une teneur en oximes 3'''-déhydrocardénolidetridigitoxosides de formule (I).

4. Composés selon la revendication 1 utilisés pour le traitement de l'insuffisance cardiaque.